Europäisches Patentamt

European Patent Office

Office européen des brevets

(19)

(11) **EP 1 591 070 A1**

(12) # EUROPEAN PATENT APPLICATION

(43) Date of publication:
 **02.11.2005 Bulletin 2005/44**

(51) Int Cl.[7]: **A61B 17/22**

(21) Application number: **05075932.3**

(22) Date of filing: **19.04.2005**

(84) Designated Contracting States:
 **AT BE BG CH CY CZ DE DK EE ES FI FR GB GR
 HU IE IS IT LI LT LU MC NL PL PT RO SE SI SK TR**
 Designated Extension States:
 **AL BA HR LV MK YU**

(30) Priority: **26.04.2004 IT MI20040811**

(71) Applicant: **Salle, Armando
 24050 Zanica BG (IT)**

(72) Inventor: **Salle, Armando
 24050 Zanica BG (IT)**

(74) Representative: **Faraggiana, Vittorio
 Ingg. Guzzi & Ravizza S.r.l.
 Via Vincenzo Monti 8
 20123 Milano (IT)**

(54) **Method and device for generation of shock waves with controlled focalization**

(57) A method of generation of focalized shock waves in which at least three shock-wave emitting units are arranged unaligned and are activated by a respective impulse with predetermined time delay relative to each other to cause the fronts of the waves emitted by the emitter units to meet in a desired focal zone. A device in accordance with the method includes at least three shock-wave emitter units (11) connected to a control device (12) that emits impulses for activation of each emitter unit with the delays necessary for positioning of the focus at the desired point in space.

Fig. 1

## Description

This invention relates to a method and a device for the generation of focalized shock waves. In particular, this invention relates to the generation of shock waves in electromedical equipment, for example of the type for treatment of some pathologies like tendonitis of the shoulder, epitrocleite, Achillean tendonitis, plantar fascitis, epicondylitis, rotulean tendinitis, calcanean spur, pubalgia, muscular contracture, traumatic and microtraumatic bursitis, cap of the rotators, simple tendinitis, plastic penis induration (scaphoid), pseudarthrosis (scarring outcome) and others.

In this equipment the shock waves can be generated in different ways. The most used are piezoelectric, electromagnetic, electrohydraulic and ballistic.

In piezoelectric systems a ceramic material with piezoelectric characteristics is subjected to an electrical impulse. The electric impulse modifies the dimension of the ceramic material to generate the desired mechanical impulse.

In electromagnetic systems an electrical impulse is circulated in a coil. The coil produces an electromagnetic field that expels a metallic membrane to produce the mechanical impulse.

In electrohydraulic systems an electrical impulse in a fluid produces gas bubbles with resulting mechanical impulse due to the pressure increase. The bubbles disappear immediately.

Lastly, in ballistic systems balls are accelerated on a guide by means of compressed air. At the end of the path, the balls have an impact that generates the shock wave.

However the shock wave is produced, the greater part of the treatments necessitate a focalized shock wave. In the focal zone, the shock waves produce much higher pressure impulses as compared with the zone outside of the focal zone.

For this purpose in the prior art mechanical focusing devices generally realized with an appropriate arrangement of surfaces reflecting the wave toward the desired focal point and/or an appropriate arrangement of the generating devices are used. For example, a mechanism for generating focalized shock waves consists of covering a concave spherical surface with piezoelectric ceramics. The waves generated by this device converge in the center of the sphere.

The greater part of the equipment concentrates the shock waves in a zone by means of a focalization mechanism that acts after generation. The most used mechanisms are elliptical reflectors (US 5.174.280) or parabolic reflectors (US 5.058.569) and ultrasound lenses (US 5.033.456).

Some of these systems have a fixed focal distance. To change the focal point position it is necessary to change the position of the emitting equipment. Others have a mechanically variable focal distance and to modify the focal distance the form of the reflector of the lenses and/or the mutual position between emitters and reflector is changed in some way.

A mechanism allowing generation of focalized shock waves has also been proposed (US 5.374.236). The mechanism consists of electromagnetic generation of waves by means of a series of concentric coils. The outermost coil generates the impulse first. A moment later, the next coil inward generates its impulse. And so forth until reaching the central coil. Allowing appropriate delay times, the effect of producing a wave with a spherical front converging towards the center of the sphere is obtained. By means of this mechanism it is also possible to change the focal distance along the straight line coaxial with the axis of the concentric coils. With this mechanism the focus can be moved only on the axis of the coils. Positioning of the focus at other points in space is possible only by moving the generating device so as to point the axis of the coil in the desired direction.

Especially in the case of treatment requiring movement of the focus around a point and/or along a predetermined path, cumbersome and relatively costly mechanical movement devices are necessary.

The general purpose of this invention is to remedy the above mentioned shortcomings by making available a shock wave generation method and a generating device in accordance with the method allowing obtaining shock waves focalized with the focal position modifiable in space with no need of moving parts. In addition, for the generation of waves focalized in accordance with the principles of this invention, mechanical focalization systems with shaped reflecting surfaces are not indispensable.

In view of this purpose it was sought to provide in accordance with this invention a method of generation of focalized shock waves in which at last three shock-wave emitting units are arranged unaligned and activated by an impulse with predetermined time delay relative to each other to cause the fronts of the waves emitted by the emitter units to meet in a desired focal zone.

Again in accordance with this invention it was sough to realize a device for generation of focalized shock waves including at least three shock-wave emitting units arranged unaligned and connected to a control device emitting impulses for activation for each emitter unit with the control device having delay means in the emission of impulses that are controlled so that the emitting units are activated by a respective impulse with predetermined temporal delay relative to each other to have the fronts of the waves emitted by the emitter units meet in a desired focus zone in space.

To clarify the explanation of the innovative principles of this invention and its advantages compared with the prior art there is described below with the aid of the annexed drawings a possible embodiment thereof by way of non-limiting example applying said principles. In the drawings:

FIG 1 shows a diagrammatic principle view of a device for generation of focalized shock waves realized in accordance with this invention, and
FIG 2 shows a diagrammatic perspective view of a possible embodiment of a generator device in accordance with this invention.

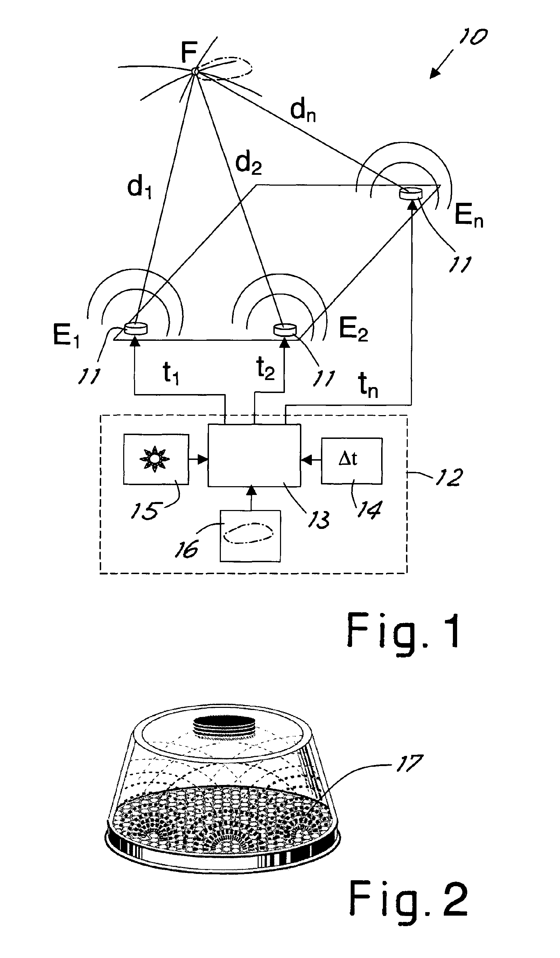

With reference to the figures, FIG 1 shows a generator device designated as a whole by reference number 10. This device includes known unfocalized wave-emitter units 11 (distinguished by $E_1$, $E_2$, ..., $E_n$ in FIG 1) advantageously realized with known ceramic piezoelectric units.

The units are at least 3 in number arranged unaligned.

Each emitter unit 11 is driven by an electrical impulse emitted by a control system 12 and therefore generates its own mechanical impulse. The control system is readily imaginable to those skilled in the art on the basis of the description of this invention given here and has delay means 13 controlled in emission of the piloting impulses. The control system can be realized in various ways. In particular, it was found advantageous to use a programmable electronic system appropriately programmed.

Upon reception of the control impulse, each emitter unit emits forward a wave with generally hemispherical front that travels in space in withdrawing from the emitter. After the generation moment, the distance of the wave front from the respective emitter depends on the propagation speed of the wave in the medium.

By definition, a focal point or zone is that point or zone in space where the shock waves produce pressure impulses substantially higher than in the zone around said focal zone.

In accordance with the method of this invention the moments $t_1$, $t_2$, ..., $t_n$ of emission of the shock wave by each emitter are such as to cause the wave fronts of the various emitters to reach the desired focusing zone virtually at the same time.

By adjusting the moment of emission it is possible to choose the focal point freely in the space in front of the series of emitters.

In other words, the moments of emission are calculated so that the respective shock waves travel the distance ($d_1$, $d_2$, ..., $d_n$) separating each emitter from the desired focus F and meet virtually in the desired focus zone.

To position the focus on a point F it is necessary therefore to calculate the delay to be given to the electrical impulse of each emitter.

One way of calculating this delay is to first calculate the distances ($d_1$, $d_2$, ..., $d_n$) of each emitter from the desired focus F. Then the emitter that proves to be furthest from the focus is selected as 'base'. Let us call this emitter 'pilot emitter'. To the latter is assigned a delay of zero by convention.

To calculate the delay of each emitter the difference between the distances of the pilot emitter from the focal point F less the distance of the emitter in question from the focal point is calculated. This distance difference is divided by the speed of the sound/ultrasound in the wave propagation medium. Division gives the delay time by which the impulse to the emitter in question must be given.

The formula for calculation of the delay to be given to the impulse for the $i^{th}$ emitter is given by the following formula:

$$\Delta t_i = \frac{(d_p - d_i)}{v}$$

where:

$d_p$ is the distance of the pilot emitter from the focal point,
$d_i$ is the distance of the $i^{th}$ emitter from the focal point, and
$v$ is the velocity of the sound/ultrasound in the propagation medium used.

It thus suffices to perform the calculation for all the emitters except the pilot and then activate the emitters in sequence with the calculated delays. Advantageously the control device includes means 14 of calculation of the delays starting from the spatial position desired for the focus.

Another advantage of this system is the fact that the waves produced can be concentrated on the focal zones of the desired dimensions.

Up to this point we have always spoken of the 'focal point'. The focal point is the one that receives the greatest pressure due to the contemporaneous nature of the arrival of the waves. Around the focal point there is a zone that receives in any case high pressure even if not as high as the focal point. For some particular purposes it might be useful that the focal zone be more extensive. Some researches signal that shock waves focalized on a more extensive zone can get the same therapeutic effects with less intense and hence more bearable pain. In addition, waves focalized on a more extensive zone also make possible therapy without the use of an X-ray or ultrasound pointing system.

For example, some research defines as 'focal zone' the zone enclosed inside the isobar whose pressure is peak pressure -6 dB (i.e. 50% less). They also define the 'treatment zone' as the zone enclosed inside the isobar of 5 MPa (50 bar) that is considered the minimum pressure for obtaining a therapeutic effect.

With the method and the device of this invention it is simple to obtain a more extensive focal zone. It suffices to modify the calculation of the delay time described above. Instead of calculating the time of each unit by always using the distances from the same focal point, for each unit or group of units calculations can be done on the basis of a slightly different focal point but always inside the focal zone. It can be decided in advance what will be the form of the focal zone and how the energy is to be distributed in the focal zone. On the basis of the desired energy distribution, one can calculate the distribution of the focal points to be used in calculating the delay times. Means 15 of setting the focal dimension can be provided so as to act appropriately on the emission delays.

The capability of modifying the focal zone can be useful in different circumstances. Non-limiting examples follow:

- it can allow positioning the focal zone in the zone where it is desired to do the therapy,
- it can allow correcting the focal zone as a consequence of movements of the patient,
- it can allow changing of the focal zone to apply the therapy on another zone, and
- it can allow scanning with the focal zone a much broader treatment zone.

All this can be done piloted by the therapist or automatically and in any case with no need of modifying the position or form of any device.

If desired, the control system can also pilot the movement of the focus along a predetermined path in real time. By way of example FIG 1 shows in dots & dashes a small path along a closed line. To do this, the control system can calculate and memorize a table of delays to be applied sequentially to the emitters to scan all the points of the path with a desired resolution. Trajectory setting means 16 are provided for this purpose.

It should be noted that with the device of this invention it is also possible to adjust the intensity of each electrical impulse and hence of each mechanical impulse separately. This can help to obtain the desired energy distribution.

Another advantage of this device is the capability of reducing the mean distance that the wave covers to the focal zone from the moment of its generation. The intensity of the wave decreases proportionately to the square of the distance covered. Hence, the shorter the path, the more pressure will reach the focal zone.

The focalization systems with lenses need sufficient distances to focalize the waves. In mechanisms based on reflectors, the waves must first go from the point of generation to the reflectors and then return to the focal zone. In addition, because of their concave shape, parabolic or elliptical reflectors have reflection points quite far from the focal zone. Devices that generate the waves directly on a concave spherical surface have all their points at the same distance from the focal zone.

For the sake of simplicity, FIG 1 shows a device with three emitters. The number of emitters and their arrangement can however be different. A larger number of emitters allows obtaining more power in the focus while keeping the power of the individual emitter limited.

In addition, a larger number of emitters can make the spatial resolution of the device more precise and thereby allow better control of the shape and arrangement of the focal zone.

In its general form, the device in accordance with this invention consists of a surface covered with a good number of emitters (for example ceramic piezoelectric) to realize an emission surface which we call here 'mosaic'.

In the device of this invention, by using for example a round flat mosaic 17 as shown for example in FIG 2 the points furthest from the focal zone are at a distance similar to those of a generator device on a concave spherical surface while the points on the central zone of the mosaic are much closer. Hence, the mean distance to the focal zone and the path of the waves will be reduced.

The mosaic can be realized with a flat shape but also with other formats. One possibility consists of realizing the mosaic in such a manner as to resemble the forms of the zone to be treated. For example, to treat a shoulder, a mosaic with concavity surrounding the shoulder can be realized. In this manner the generating points will be very close to the desired focal zone and in addition it will be possible to move the focal zone onto different zones of the shoulder without changing the position of the device. Even the individual emitters or groups of emitters can be all the same or have different shapes and dimensions.

Advantageously, a first mechanical focalization of the shock wave can be obtained by arranging the emitters according to a surface provided with a focus and having preferably a spherical or parabolic shape. Then, the first focalization of the shock wave can be oriented on the plane or deeply thanks to the control of the emitters with a differential delay according to the method of this invention. This can be helpful, for example, in making control easier and/or increasing the energy efficiency of the system.

Naturally the above description of an embodiment applying the innovative principles of this invention is given by way of non-limiting example of said principles within the scope of the exclusive right claimed here. For example, a computerized interface with a monitor for viewing the parameters and a keyboard for setting the parameters could be easily provided.

**Claims**

1. Method for generation of focalized shock waves in which at least three shock-wave emitting units are arranged unaligned and activated by a respective impulse with predetermined time delay relative to each other to cause the fronts of the waves emitted by the emitter units to meet in a desired focal zone.

2. Method in accordance with claim 1 in which after defining a pilot emitter unit to which to assign by convention zero delay, the delay to be given to the impulse for the ith emitter is calculated with the following formula:

$$\Delta t_i = \frac{(d_p - d_i)}{v}$$

where:

$d_p$ is the distance of the pilot emitter from the focal point,
$d_i$ is the distance of the ith emitter from the focal point, and
$v$ is the velocity of the sound/ultrasound in the propagation medium used.

**3.** Method in accordance with claim 2 in which the emitter unit chosen as pilot is the one at the greatest distance from the desired focus.

**4.** Method in accordance with claim 1 in which the amplitude of the focus zone is regulated by varying the emission delay of the emitters.

**5.** Method in accordance with claim 1 in which the focus is moved in real time in the space along the predetermined path by varying the emission delay of the emitters.

**6.** Method in accordance with claim 1 in which the emitters are a plurality arranged to form a mosaic on a surface.

**7.** Method in accordance with claim 1 in which the emitters are piezoelectric emitters.

**8.** Device for generation of focalized shock waves including at least three shock-wave emitting units arranged unaligned and connected to a control device emitting impulses for activation for each emitter unit with the control device having delay means (13) in the emission of impulses that are controlled so that the emitting units are activated by a respective impulse with predetermined temporal delay relative to each other to have the fronts of the waves emitted by the emitter units meet in a desired focus zone in space.

**9.** Device in accordance with claim 8 **characterized in that** it includes calculation means (14) that, after defining a pilot emitter unit to which zero delay is assigned conventionally, calculate the delay to give to the impulse for the ith emitter based on the following formula:

$$\Delta t_i = \frac{(d_p - d_i)}{v}$$

where:

$d_p$ is the distance of the pilot emitter from the focal point,
$d_i$ is the distance of the ith emitter from the focal point, and
$v$ is the velocity of the sound/ultrasound in the propagation medium used.

**10.** Device in accordance with claim 8 **characterized in that** it includes means (15) of varying the emission delay of the emitters to regulate the amplitude of the focus zone.

**11.** Device in accordance with claim 8 **characterized in that** it includes means (16) of varying the emission delay of the emitters to move the focus in the space along a determined path.

**12.** Device in accordance with claim 8 **characterized in that** the emitters are a plurality arranged to form a mosaic (17) on a surface.

**13.** Device in accordance with claim 8 **characterized in that** the emitters are piezoelectric emitters.

**14.** Device in accordance with claim 8 **characterized in that** the emitters are arranged according to a surface provided with a focus and having preferably a spherical or parabolic shape.

Fig. 1

Fig. 2

| European Patent Office | EUROPEAN SEARCH REPORT | Application Number EP 05 07 5932 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 860 004 B (THE BOARD OF REGENTS ACTING FOR AND ON BEHALF OF THE UNIVERSITY OF MIC) 8 January 2003 (2003-01-08)<br>* column 6, line 9 - column 7, line 8 *<br>* figure 2 *<br>----- | 1-13 | A61B17/22 |
| X | US 6 042 556 A (BEACH ET AL)<br>28 March 2000 (2000-03-28)<br>* column 5, line 54 - column 6, line 52 *<br>* column 10, line 33 - line 61; figures 1-5 *<br>----- | 1-13 | |
| X | US 5 316 000 A (CHAPELON ET AL)<br>31 May 1994 (1994-05-31)<br>* the whole document *<br>----- | 1-13 | |
| X | US 6 428 477 B1 (MASON MARTIN K)<br>6 August 2002 (2002-08-06)<br>* column 6, line 54 - column 8, line 59; figures *<br>----- | 1-13 | |
| | | | TECHNICAL FIELDS SEARCHED (Int.Cl.7)<br><br>A61B |

The present search report has been drawn up for all claims

| Place of search | Date of completion of the search | Examiner |
|---|---|---|
| Munich | 17 August 2005 | Held, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 05 07 5932

This annex lists the patent family members relating to the patent documents cited in  the above-mentioned European search report.
The members are as contained in the European Patent Office EDP file on
The European Patent Office is in no way liable for these particulars which are merely  given for the purpose of information.

17-08-2005

| Patent document cited in search report | | Publication date | Patent family member(s) | | Publication date |
|---|---|---|---|---|---|
| EP 0860004 | B | 26-08-1998 | US | 5590657 A | 07-01-1997 |
| | | | AU | 7466896 A | 29-05-1997 |
| | | | DE | 69625700 D1 | 13-02-2003 |
| | | | DE | 69625700 T2 | 18-09-2003 |
| | | | EP | 0860004 A1 | 26-08-1998 |
| | | | JP | 2000500038 T | 11-01-2000 |
| | | | WO | 9717693 A1 | 15-05-1997 |
| US 6042556 | A | 28-03-2000 | AU | 6133099 A | 27-03-2000 |
| | | | WO | 0013598 A1 | 16-03-2000 |
| US 5316000 | A | 31-05-1994 | FR | 2673542 A1 | 11-09-1992 |
| | | | FR | 2679125 A1 | 22-01-1993 |
| | | | AT | 230954 T | 15-02-2003 |
| | | | AT | 226417 T | 15-11-2002 |
| | | | CA | 2105401 A1 | 06-09-1992 |
| | | | DE | 69232831 D1 | 28-11-2002 |
| | | | DE | 69232831 T2 | 18-06-2003 |
| | | | DE | 69232901 D1 | 20-02-2003 |
| | | | DE | 69232901 T2 | 13-11-2003 |
| | | | WO | 9215253 A1 | 17-09-1992 |
| | | | EP | 1034745 A1 | 13-09-2000 |
| | | | EP | 0575363 A1 | 29-12-1993 |
| | | | JP | 3368241 B2 | 20-01-2003 |
| | | | JP | 2000139941 A | 23-05-2000 |
| | | | JP | 6509241 T | 20-10-1994 |
| | | | JP | 2003126111 A | 07-05-2003 |
| | | | US | 5474071 A | 12-12-1995 |
| | | | US | 5666954 A | 16-09-1997 |
| | | | DE | 69227688 D1 | 07-01-1999 |
| | | | DE | 69227688 T2 | 01-07-1999 |
| | | | WO | 9301752 A1 | 04-02-1993 |
| | | | EP | 0595849 A1 | 11-05-1994 |
| | | | IL | 102516 A | 31-01-1996 |
| US 6428477 | B1 | 06-08-2002 | NONE | | |

EPO FORM P0459

For more details about this annex : see Official Journal of the European Patent Office, No. 12/82